# EUROPEAN PATENT SPECIFICATION

(11) **EP 1 504 765 B1**
(45) Date of publication and mention of the grant of the patent: **22.07.2015**
(21) Application number: 03723367.3
(22) Date of filing: 14.05.2003
(51) Int. Cl.: A61K 9/00, A61K 47/26, A61K 47/38, A61K 9/70

(54) **QUICKLY SOLUBLE FILM PREPARATIONS**
SCHNELLLÖSLICHE FILMPRÄPARATE
PREPARATIONS DE FILM RAPIDEMENT SOLUBLE

(30) Priority: 16.05.2002 JP 2002142196
(43) Date of publication of application: 09.02.2005
(73) Proprietor: KYUKYU PHARMACEUTICAL CO., LTD., Chuo-ku, Tokyo 103-0023 (JP)
(72) Inventor: YASUDA, Kayo, Toyama 939-0351 (JP); OKUBO, Tadatoshi, Toyama 939-0351 (JP); SAWAI, Yoshihiro, Toyama 939-0351 (JP)
(74) Representative: Von Kreisler Selting Werner - Partnerschaft von Patentanwälten und Rechtsanwälten mbB
(86) International application number: PCT/JP2003/005986
(87) International publication number: WO 2003/097103

(56) References cited:
- EP-A1- 1 008 343
- WO-A-01/34121
- WO-A-87/02241
- WO-A1-01/35934
- WO-A1-88/08298
- GB-A- 1 510 999
- JP-A- 51 054 917
- US-A- 4 126 502

## Description

### Technical Field

The present invention relates to an intraoral soluble type film-shaped preparation which contains a drug useful for treatment of a disease or diagnosis, and can be dosed without water. More particularly, a preparation of the present invention relates to an intraoral soluble type film-shaped preparation which contains a drug and an edible polymer, and is rapidly (within about 60 seconds) dissolved by intraoral water when intraorally administered.

### Background Art

As dosage forms intraorally administered, there have hitherto been known tablets, chewable tablets, buccal tablets, capsules, pills, lozenges, aqueous drugs, intraoral soluble type preparations including intraoral mucous membraneadhesive type preparations, and the like. Of these, the tablets are most widely utilized, and the capsules, the aqueous drugs and the like are also widely utilized.

As for the intraoral soluble type preparations, ones as shown below have been generally known. (1) A preparation comprising a sheet-shaped edible molded product in which an excipient, a plasticizer and a binder are blended, the preparation being obtained by mixing food materials of the excipient, the plasticizer and the binder, and/or a drug, and extruding the resulting mixture from a screw extruder, wherein the extrusion thickness thereof is from 0.1 to 5 mm, and preferably from 0.5 to 3 mm (for example, refer to patent document 1). (2) A preparation comprising a sheet-shaped administration formation to be individually administered, such as medicine, confectionery, other food or a cosmetic, which is orally administered or incorporated with a material comprising 20 to 60% by weight of a film forming agent, 2 to 40% by weight of a gel forming agent, 0.1 to 35% by weight of an active substance and further less than 40% by weight of an inactive filler applied onto a carrier or without a support, wherein the layer thickness of a layer of the material is from 0.003 to 4 mm, preferably from 20 to 400 µm, and particularly preferably from 70 to 150 µm, and the material is spread or extruded as a production method and completely decomposes in the mouth within 10 minutes (for example, refer to patent document 2). (3) A preparation which is a foil-shaped drug obtained by dissolving or suspending an active substance and/or a separating agent, mixing a foil forming agent and a filler in some cases, conducting homogenization in some cases, applying the solution or the suspension onto a foil forming machine in a foil form, and dividing a foil obtained by drying the applied material into arbitrary small pieces (units), wherein the thickness of the dried foil is from about 0.1 to 2 mm, and advantageously from 0.07 to 0.3 mm (for example, refer to patent document 3).

### Prior Art

[Patent Document 1]
   Toku-Kai-Hei (Japanese Patent Unexamined Publication) 10-179045 (page 2, left column and page 3, right column, paragraph [0016])
[Patent Document 2]
   Toku-Kai-Hei (Japanese Patent Unexamined Publication) 7-100186 (page 2 and page 4, right column, paragraph [0035])
[Patent Document 3]
   Toku-Kai-Sho (Japanese Patent Unexamined Publication) 51-29218 (page 2 and page 3) corresponding to GB-A-1510999

The above-mentioned preparation of patent document 1 (Toku-Kai-Hei 10-179045) which is the intraoral soluble type preparation is one obtained by extrusion molding. Accordingly, the preparation is produced with a single-screw extruder as shown in an example thereof, so that the film thickness becomes as thick as 1 mm or 2 mm. It takes a long time for dissolution, and it is conceivable that dissolution in the oral cavity within 60 seconds is impossible. In addition, this thickness introduces a foreign-body sensation as a preparation.

This preparation is produced with a screw extruder, and the preparation produced with the screw extruder inevitably necessitates an antiblocking agent (starch is used in this cited document) (when no antiblocking agent is used, products adheres to each other). It is conceivable that this is caused by characteristics of a polymer used for easily dissolving the preparation with a small amount of water, which increases the number of processes to cause fear of high cost. Further, particularly in the case of a preparation containing a drug or another diagnostic agent, visual value thereof is important, and it is likely to lower it by the antiblocking agent.

Further, the preparation produced in patent document 1 with the screw extruder is subjected to aging at a relatively low temperature (20°C) for 12 hours. In the case of this production method, no drying process is required, so that there is the advantage that it becomes possible to use a material having low heat stability. However, it takes a very long time of 12 hours, so that the production efficiency is also inferior.

The above-mentioned preparation of patent document 2 (Toku-Kai-Hei 7-100186) is produced by various methods, and the thickness thereof is from 0.003 to 4 mm. By the way, a drug is produced with a plunger type extruder in the examples. In the case of the extrusion method, the thickness thereof will tend to increase, and there is the disadvantage that the dissolution time thereof is long, as described that it takes 10 minutes for dissolution. Too long the dissolution time unfavorably results in continuation of a foreign-body sensation.

Ones enumerated in this patent document 2 as a film forming agent are ones generally used as an excipient, a plasticizer, a disintegrant, a solubilizing agent or the like, and hard to be said to have film forming ability. If anything, polymers having film forming ability are found in ones used as a gel forming agent in patent document 2. However, the compounding amount of the gel forming agent is small (particularly, refer to Example 2), so that it is conceivable that this gel forming agent does not exhibit film forming ability.

For example, polyethylene glycol used as the film forming agent in Example 2 of this patent document 2 is in a solid form at room temperature, and this coagulability at room temperature forms a sheet-shaped formation having a thickness of 1 mm. This sheet-shaped formation can be expected to have a certain degree of solubility at a temperature in the oral cavity. However, the strength thereof can not be expected so much, because of utilization of coagulability at room temperature. In fact, according to the description of this patent document 2, it is described that a certain degree of strength is necessary in production (paragraph number [0017]), and the thickness of the preparation increases more and more. It is conceivable that the dissolution time is more prolonged because of this thickness. Moreover, the drug content is as low as 3.75% by weight, as described in Example 2, so that there is a limitation on the kind of drug which can be contained, which causes lack of availability.

Further, the preparation of patent document 3 (Toku-Kai-Sho 51-29218, corresponding to GB-A-1510999) contains a foil forming agent which is described to form a foil, in an amount of 6 to 20% in a solution or a suspension, and the ratio thereof is low. No film strength is described in this patent document 3, and when the foil forming agent is contained at such a low ratio, the breaking strength and tensile strength of the film formed are low, resulting in a brittle product hard to handle. Moreover, the preparation does not have "rapid solubility".

WO 01/34121 relates to a multi-layer preparation in film form consisting of hydrophilic polymers, for the rapid release of active ingredients. The preparation is characterized in that adjacent layers are different from one another, that the one of these respective layers is soluble in a non-aqueous solvent, in which the respective adjacent layer cannot be dissolved or can only be dissolved with difficulty.

As described above, the conventional intraoral soluble type preparations have disadvantages such as too thick the thickness, the foreign-body sensation, the low strength and brittleness, and the low drug content. From these, the appearance of a thin, film-shaped preparation has been desired which is easily soluble in the mouth and high in availability. That is to say, the appearance of an intraoral soluble type film-shaped preparation has been desired which is easily soluble by water in the oral cavity, and in which a drug is rapidly absorbed into the digestive tract or the oral mucous membrane and the drug content can be increased as needed.

### Disclosure of the Invention

In order to solve the above-mentioned problems, that is to say, in order to provide a film-shaped preparation which is easily soluble by intraoral water, the present inventors have made intensive studies.

That is to say, the inventors have obtained the findings that the following are necessary as preparation characteristics demanded for a rapidly soluble film-shaped preparation.
(1) To be rapidly soluble, that is to say, to be soluble in the mouth within about 60 seconds;
(2) To have an easy-to-use dosage form, that is to say, to hold such a strength that the preparation is not damaged when taken, not to generate unpleasant feeling in the oral cavity caused by too strong its strength, not to bring about inconvenient handling caused by too thin the preparation, and to have such a preparation size that it is easily taken; and
(3) To be wide in allowability of a drug applied, that is to say, to be high in the limit value of the drug content, and to be able to comply with physicochemical properties of the drug.

Then, in order to make the above-described (1) possible, the thickness of the preparation should be decreased. However, the limit value of the drug content referred in the above-described (3) decreases to several milligrams thereby, and the bad influence caused by the thinning also appears, resulting in the possibility of failing to satisfy the feature of the above-described (2).

In order to make the feature of (2) possible, a certain degree of strength is required, so that usually, the compounding ratio of a polymer is set relatively high, or a polymer having a relatively high polymerization degree is used. As a result, the conditions in (1) and/or (3) may not be satisfied. An increase in the amount of the polymer makes it difficult to dissolve, and generates unpleasant feeling in the oral cavity.

Among the characteristics of (1) through (3), it is important for popularizing the film-shaped preparation that the limit value of the drug referred in (3) can be set high. In order to satisfy this, the compounding ratio of the drug is set high, or the preparation size is enlarged. However, it becomes impossible to maintain the strength by setting the compounding ratio of the drug high, or too large the preparation size causes inconvenience when the preparation is taken, resulting in the possibility of failing to satisfy the conditions of (2). Further, it is also conceivable to increase the thickness of the preparation, but as a result, the conditions of (1) is not satisfied.

Considering the above-described conditions, studies have been made. As a result, it has been found that one is suitable in which the breaking strength of a film is from 200 to 3,000 g/φ7 mm (measured with a FUDOH rheometer described below), and the tensile strength of the film is from 200 to 3, 000 g/15 mm (measured with the FUDOH rheometer described below).

Further, it has also been found that the film is required to have a thickness of 30 µm or more, taking into consideration the avoidance of inconvenient handling caused by too thin the preparation, and that a thickness of 300 µm or less is suitable, taking into consideration such a preparation size that the preparation is easily taken.

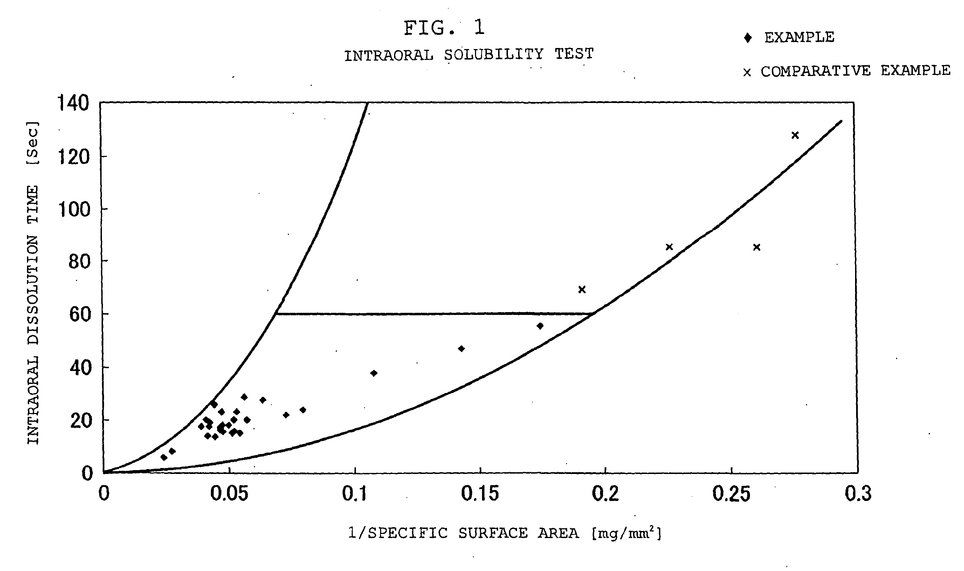

Film-shaped preparations in which the breaking strength of the above-mentioned film was from 200 to 3, 000 g/φ7 mm, and the tensile strength of the film was from 200 to 3,000 g/15 mm, and ones exceeding these ranges were prepared as shown in Examples and Comparative Examples described below. Then, the intraoral dissolution time [sec] thereof was plotted as Y, and the reciprocal [mg/mm²] of the specific surface area [mm²/mg] as X. As a result, surprisingly, a graph of Fig. 1 showing the relationship between the intraoral dissolution time of the film preparation and the reciprocal of the specific surface area of the film was obtained. That is to say, in all the film-shaped preparations in which the breaking strength of the film was from 200 to 3,000 g/φ7 mm, and the tensile strength of the film was from 200 to 3,000 g/15 mm, the intraoral dissolution time thereof was within 60 seconds, and rapidly soluble film-shaped preparations demanded in this application were obtained.

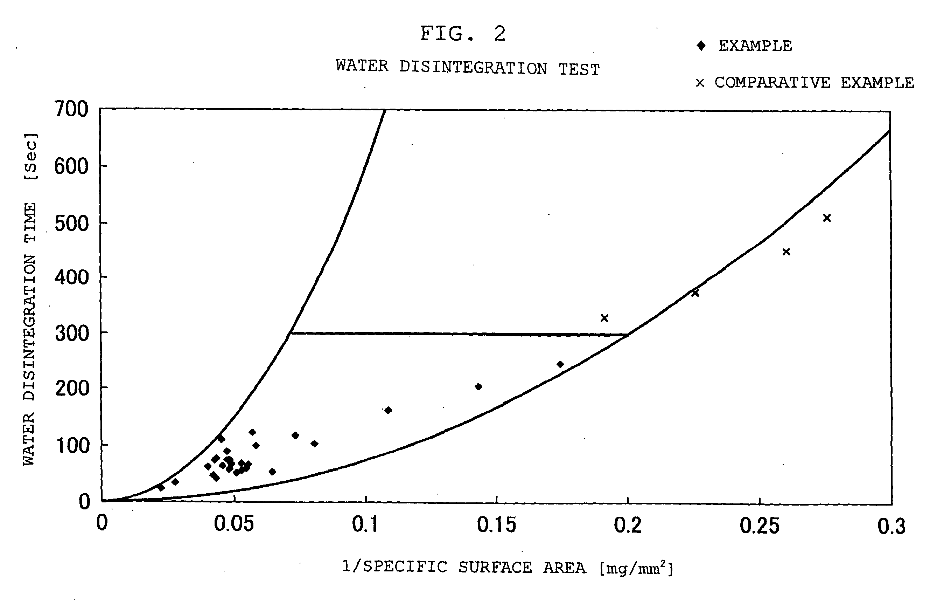

The present inventors have further studied the above-mentioned conditions. In place of the above-mentioned intraoral dissolution time of the film preparation, the water disintegration time (a water disintegration test is as described below) was plotted as Y, and the reciprocal [mg/mm²] of the specific surface area [mm²/mg] as X. As a result, a graph of Fig. 2 showing the relationship between the water disintegration time of the film preparation and the reciprocal of the specific surface area of the film was obtained. It was also found that an intraoral dissolution time of within 60 seconds in Fig. 1 corresponded to a water disintegration time of within 300 seconds in Fig. 2.

The present invention has been achieved on the finding that the water disintegration time (Y) correlates with the reciprocal (X) of the specific surface area [mm²/mg] of the film, which has not hitherto been known at all, and the gist thereof is as follows.

That is to say, the present invention relates to (1) a two- or three-layered film preparation comprising a drug-containing layer and a support layer on one or both side(s) of the drug-containing layer,
wherein both the drug-containing layer and the support layer(s) contain an edible polymer,
wherein the edible polymer is hydroxypropyl cellulose and/or hydroxypropylmethyl cellulose,
wherein the compounding ratio of the drug is from 0.01 to 40% by weight, based on the whole preparation, and the compounding ratio of the edible polymer is from 40 to 99.99% by weight, based on the whole preparation,
wherein the film-shaped preparation has a thickness of from 30 µm to 300 µm, and
wherein the breaking strength of the multi-layered film preparation is from 200 to 3,000 g/φ7 mm, the tensile strength of the film is from 200 to 3,000 g/15 mm, and the preparation is soluble in the oral cavity within 60 seconds;
(2) The film preparation according to (1),
   wherein the edible polymer of the support layer is hydroxypropylmethyl cellulose, the compounding ratio thereof is 50% by weight or more, the edible polymer of the drug-containing layer is hydroxypropyl cellulose, and the compounding ratio thereof is from 40 to 99.99% by weight;
(3) The film preparation according to (1) or (2), wherein the drug is contained to provide a dose of 0.001 mg to 50 mg, and soluble or dispersible in a water-ethanol solvent;
(4) The film preparation according to (1), (2) or (3), which further contains a saccharide;
(5) The film preparation according to (4), wherein the sum of the drug and the saccharide is from 25 to 40% by weight based on the whole preparation.

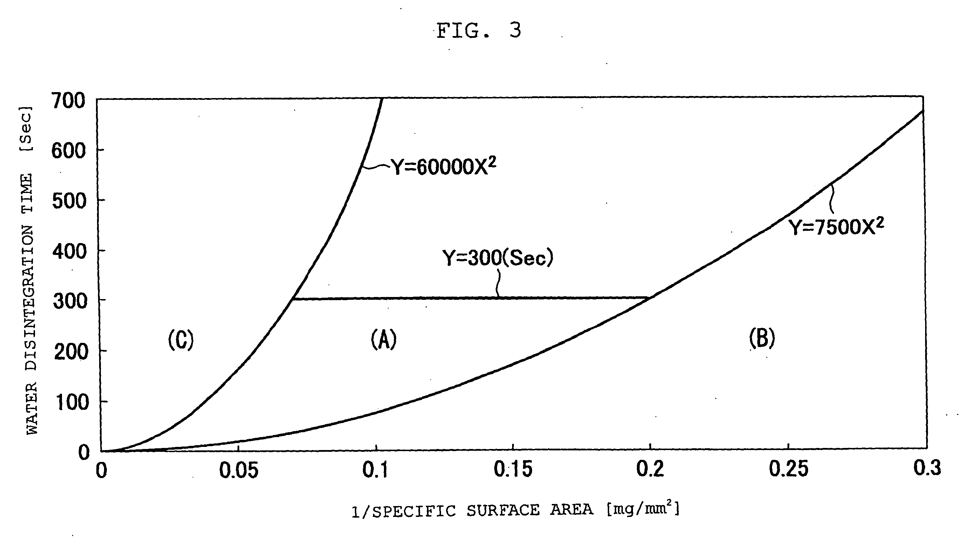

In the above-described, "the water disintegration time Y [sec] is within 300 seconds" has the same meaning as "soluble in the oral cavity within 60 seconds" as explained abbove in detail, and racial differences and differences among individuals can be removed to keep objectivity. Further, in water disintegration time Y [sec]<7500X² (a region of (B) in a graph of Fig. 3 showing the relationship between the water disintegration time of the film preparation and the reciprocal of the specific surface area of the film), the dissolution of the film preparation is rapid. However, the desired strength is not kept. In the film preparation of the present invention, the thickness thereof is suitably from 30 µm to 300 µm.

When the breaking strength is lower than 200 g/φ7 mm, the product unfavorably becomes brittle to cause difficulty in handling. On the other hand, higher than 3, 000 g/φ7 mm results in the generation of unpleasant feeling when the preparation is kept in the mouth.

When the tensile strength is lower than 200 g/15 mm, the product unfavorably becomes brittle to cause difficulty in handling. On the other hand, higher than 3, 000 g/15 mm results in the generation of unpleasant feeling when the preparation is kept in the mouth.

When the breaking strength is higher than 3,000 g/φ7 mm, or the tensile strength is higher than 3,000 g/15 mm, there is a fear of a prolonged dissolution time. It is therefore unfavorable.

One unit film preparation of the present invention can contain as much as 50 mg of the drug as needed. For example, when the minimum total weight and dimension of the film preparation of the present invention containing 50 mg of the drug are shown, the total weight is 125 mg, and the minimum dimension is 28 mm×20 mm×190 µm.

When the film-shaped preparation is not formed by one layer, a support layer containing an edible polymer may be provided on one side or both sides of the drug-containing layer. When the compounding ratio of the drug is set high (for example, from 25 to 40% by weight) in one layer, the strength does not reach a required strength in some cases. However, when the support layer containing the edible polymer is provided on one side or both sides of the drug-containing layer, it becomes possible to satisfy all the preparation characteristics demanded for the rapidly soluble film-shaped preparation, and it becomes possible to avoid blocking (adhesion) of the preparation films to each other. Further, it becomes possible to improve visual value dramatically. Accordingly, not only a merit of increasing the strength of the preparation, but also a synergistic function can be expected.

Hydroxypropylmethyl cellulose is an easily water-soluble edible polymer, hard to be affected by the outside air moisture, lustrous when used in the film preparation, and relatively hard to decrease in strength even when titanium oxide or a colorant is added. Accordingly, it is suitable for the support layer.

On the other hand, hydroxypropyl cellulose is soluble in the whole range from water to 100% ethanol, and has the advantage that the soluble range can be selected from the wide range from water to 100% ethanol to the drug.

Further, as a preferred embodiment, 50% by weight or more of hydroxypropylmethyl cellulose, 10 to 30% of a saccharide and 5 to 20% of a plasticizer, and 15% by weight or less of titanium oxide or 5.0% or less of a colorant are incorporated in the support layer, and 40 to 99.99% by weight of hydroxypropyl cellulose and the drug are essentially incorporated in the drug layer, and a saccharide or/and a plasticizer are incorporated as needed.

The saccharide may be, of course, mixed with the drug. Water solubility and water disintegratability are improved thereby. In particular, the saccharide is suitable as one for improving water solubility and water disintegratability also in that it does not affect the properties of the drug. When the preparation is divided into the drug layer and the support layer, the saccharide is preferably contained in only the support layer or in the support layer in larger amounts.

One in which the water disintegration time Y [sec] satisfies the relationship of 60000X²≥Y≥7500X² corresponds to a region of (A) in the graph of Fig. 3. The meaning of 60000X²≥water disintegration time Y [sec] indicates a region in which the drug can be contained in relatively large amounts, and the range of 60000X²<Y (a region of (C) in the graph of Fig. 3) shows a state in which the drug can be contained only in extremely small amounts.

In the film-shaped preparation of the present invention the thickness thereof, as long as the braking strength and tensile strength thereof are within the above-mentioned ranges is from 30 to 300 µm, taking into consideration ease of handling, unpleasant feeling at the time when the preparation is taken and the like, and further the dissolution time.

The film-shaped preparation of the present invention is soluble in the oral cavity within approximately 60 seconds, and easily taken with a reduced unpleasant feeling. It is therefore advantageous. Further, it is soluble within such a short period of time, whereby the drug is rapidly absorbed into the digestive tract or the oral mucous membrane.

The film preparation of the present invention is rapidly (within about 60 seconds) soluble in the oral cavity, and the drug is rapidly absorbed into the digestive tract or the oral mucous membrane by physicochemical properties thereof. Thus, the function of the drug is instantly appeared. In particular, for the aged whose amount of saliva is small, even when the preparation is taken without drinking of water, it is easily soluble in the oral cavity.

Accordingly, the film preparation of the present invention is expected to have a pharmacologic effect similar to that of conventional oral preparations such as tablets and capsules, and can be easily taken without water. It has therefore the advantage that it can be safely administered even to the aged and children.

### Brief Description of the Drawings

Fig. 1 is a graph showing the relationship between the intraoral dissolution time of a rapidly soluble film preparation of the present invention and the reciprocal [mg/mm²] of the specific surface area [mm²/mg] of a film, and Fig. 2 is a graph showing the relationship between the water disintegration time of a rapidly soluble film preparation of the present invention and the reciprocal [mg/mm²] of the specific surface area [mm²/mg] of a film.
Fig. 3 is a graph showing a region to be satisfied by a rapidly soluble film preparation of the present invention, in the graph showing the relationship between the water disintegration time of the film preparation and the reciprocal of the specific surface area [mm²/mg] of the film.

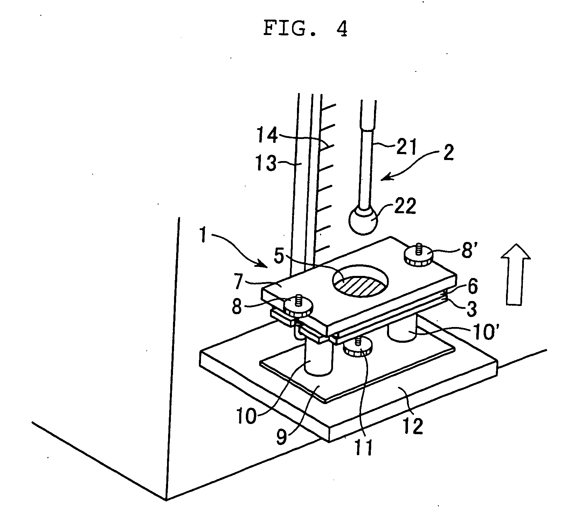

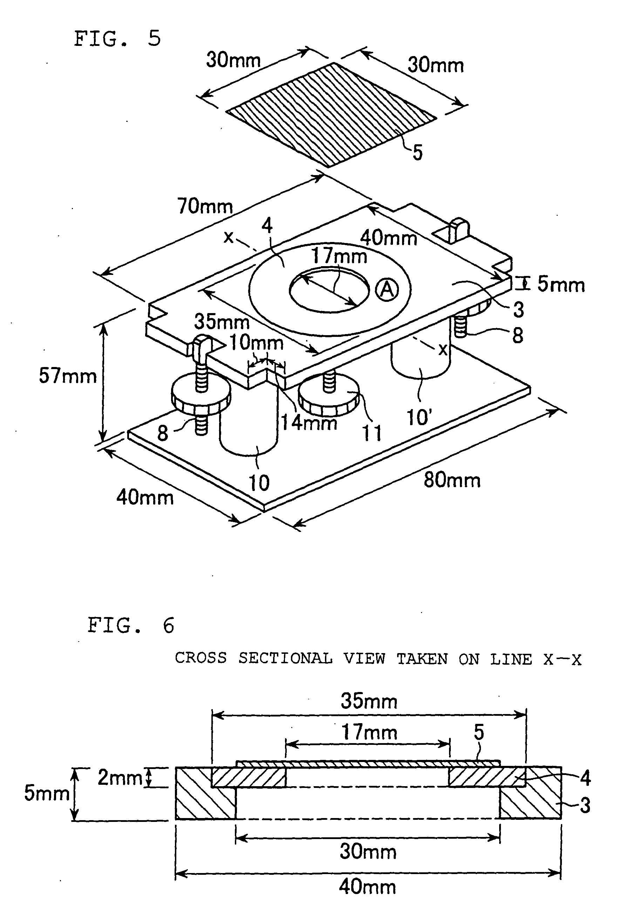

Fig 4 is a schematic perspective view showing a breaking strength measuring apparatus used in the present invention, Fig. 5 is a schematic perspective view showing the shape and size of a part of an adapter for braking stress in the breaking strength measuring apparatus, and Fig. 6 is a cross sectional view taken on line X-X, showing the cross sectional shape and size of a lower plate for fixing a test piece of the adapter for braking stress in the breaking strength measuring apparatus, a ring placed on the plate and the test piece.

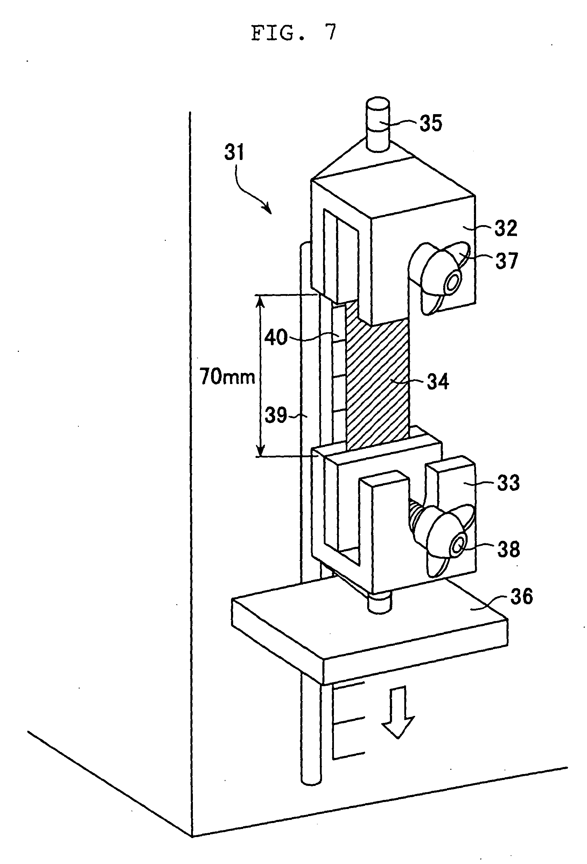

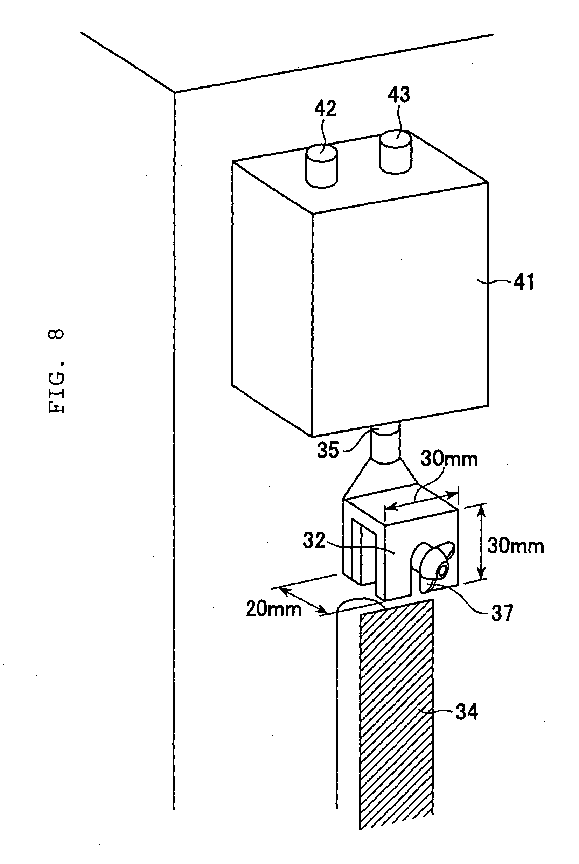

Fig. 7 is a schematic perspective view showing a tensile strength measuring adapter of a tensile strength measuring apparatus used in the present invention, and Fig. 8 is a schematic perspective view showing a detecting unit of the tensile strength measuring apparatus, to which an upper adapter is attached.

### [Description of Reference Numerals and Signs]

1. Adapter for Breaking Stress
2. Adapter for Viscosity
3. Lower Plate for Fixing Test Piece
4. Ring
5. Test Piece
6. Intermediate Plate for Fixing Test Piece
7. Upper Plate for Fixing Test Piece
8, 8'. Set Screws
9. Base Plate
10, 10'. Support Rods
11. Screw for Fixing Adapter
12. Test Desk
13. Test Desk Up-and-Down Movement Slit
14. Equally Spaced Scale for Moving Velocity
21. Rod-Like Body
22. Sphere-Like Body
31. Tensile Strength Measuring Adapter of Tensile Strength Measuring Apparatus
32. Upper Adapter for Fixing Test Piece
33. Lower Adapter for Fixing Test Piece
34. Test Piece
35. Upper Adapter Attaching Member
36. Test Desk
37. Upper Adapter Set Screw
38. Lower Adapter Set Screw
39. Test Desk Up-and-Down Movement Slit
40. Equally Spaced Scale for Moving Velocity
41. Detecting Unit
42. Knob for Coarse Adjustment of Indicator
43. Knob for Fine Adjustment of Indicator

### Best Mode for Carrying Out the Invention

The present invention is described in more detail below.

The film preparation of the present invention is a two or three-layer structure in which a support layer is provided on one side or both sides of the drug layer. The drug layer of the present invention contains the drug and an edible polymer, and a saccharide, a plasticizer and the like if necessary. The support layer contains an edible polymer, and a saccharide, a plasticizer and the like if necessary. Either of the two- or three-layer structure of the drug layer and the support layer is soluble in the oral cavity within approximately 60 seconds, as long as the thickness is adjusted to 30 to 300 µm, the breaking strength thereof is from 200 to 3, 000 g/φ7 mm, and the tensile strength is from 200 to 3,000 g/15 mm, and the drug is transmitted toward the digestive tract. The preparation is easy to be handled, and does not have unpleasant feeling when taken. It is therefore advantageous.

With respect to the difference according to the presence of absence of the support layer, when the support layer is provided, the effect is obtained that a definite strength is easily maintained while keeping high solubility in the oral cavity. Further, blocking of the preparation can be avoided, and the visual value of the preparation can be improved.

The edible polymers used in the present invention include hydroxypropyl cellulose (HPC) and/or hydroxypropylmethyl cellulose (HPMC).

HPC and HPMC are suitably used. The saccharides used, if necessary, include maltose, hydrogenated maltose starch syrup , maltitol, erythritol, xylitol, sucrose, sorbitol and the like. Maltose, maltitol and hydrogenated maltose starch syrup are suitably used. The plasticizers similarly used, if necessary, include polyethylene glycol (PEG), glycerol, sorbitol and the like. PEG-400 and PEG-4000 are suitably used. Further, a sweetener such as saccharin sodium, aspartame, acesulfame potassium or sucrarose is used if necessary. Further, the use of various flavors, colorants such as titanium oxide and various dyes is, of course, permissible.

The drug used in the present invention is preferably one given in a dose of 0.001 mg to 50 mg, and there is no particular limitation thereon as long as the drug is soluble or dispersible in a water-ethanol solvent. The advantage of the rapidly soluble film preparation of the present invention is essentially that the preparation can be taken without drinking of water. Accordingly, the drug which exerts no adverse effect on the digestive organs and the like even in such a case is desirable. Such drugs include, for example, drugs as described below.

Minor tranquilizers such as alprazolam, fludiazepam and lorazepam, hypnagogic agents such as zolpidem tartrate, antiparkinson agents such as cabergoline and methixene hydrochloride, Alzheimer type dementia treating agents such as donepezil hydrochloride, arthrifuges such as colchicines, bronchodilators such as clenbuterol hydrochloride, salbutamol hydrosulfate, fenoterol hydrobromide and procaterol hydrochloride, antiulcer agents such as rabeprazol Na, famotidine and lafutidine, antidiabetic drugs such as voglibose, antiarrhythmic agents such as indenolol hydrochloride and bufetolol hydrochloride, antihypertensive agents such as enalapril maleate, quinapril hydrochloride, cilazapril, nifedipine, felodipine and benidipine hydrochloride, lipid-lowering agents such as simvastatin, vitamin preparations such as thiamin hydrochloride and hydroxocobalamin hydrochloride, immunosuppressive agents·atopic dermatitis treating agents such as tacrolimus hydrate, hormone preparations such as ethynyl estradiol·methyl estrenolone, migraine headache treating agents such as lomerizine hydrochloride, antidinics such as ifenprodil tartrate, antitussive drugs such as chloperastine and clofedanol hydrochloride, antidiarrheal drugs such as loperamide hydrochloride, cathartics such as Na picosulfate, antiemetic drugs such as azasetron hydrochloride, granisetron hydrochloride and ramosetron hydrochloride, antihistamic agents such as mequitazine, homochlorcyclizine hydrochloride and chlorpheniramine maleate, antiallergic agents such as cetirizine hydrochloride and emedastine fumarate, opium alkaloid-based analgesic antitussive drugs such as ethylmorphine hydrochloride and morphine hydrochloride, and the like.

In the present invention, the compounding ratio of the drug is from 0.01 to 40% by weight based on the whole preparation, that of the edible polymer is from 40 to 99.99% by weight identically, that of the saccharide is from 0 to 60% by weight identically, that of the plasticizer is from 0 to 20% by weight identically, that of the colorant is from 0 to 10% by weight identically, and that of the flavor is from 0 to 1.0% by weight identically. When the support layer is provided in the film preparation of the present invention, the thickness of the support layer is suitably from about 5 to 40 µm, and the thickness of the drug layer is suitably from 15 to 290 µm. The whole thickness is from 30 to 300 µm as described above, more suitably from 35 to 160 µm, and still more suitably from 35 to 130 µm. Either in the case of only the drug layer or in the case of the drug layer and the support layer, a thickness exceeding 300 µm is unfavorable, because of unpleasant feeling at the time when the preparation is taken. Thinner than 30 µm is unfavorable, because the preparation becomes hard to handle, and the content of a principal agent is also limited.

Measurement of the breaking strength of the film of the film-shaped preparation of the present invention and the tensile strength of the film, and a water disintegration test and an intraoral dissolution test were conducted according to the following methods:

### [Test Methods]

### 1) [Breaking Strength]

(1) A rheometer (manufactured by Rheotech Co., Ltd., Type RT-3020D-CW) was used as a breaking strength measuring apparatus. Fig 4 is a schematic perspective view showing the breaking strength measuring apparatus used in the present invention, Fig. 5 is a schematic perspective view showing the shape and size of a part of an adapter for braking stress in the breaking strength measuring apparatus, and Fig. 6 is a cross sectional view taken on line X-X, showing the cross sectional shape and size of a lower plate for fixing a test piece in the adapter for braking stress in the breaking strength measuring apparatus, a ring placed on the plate and the test piece. In the figures, reference numeral 1 is an adapter for breaking stress, reference numeral 2 is an adapter for viscosity, reference numeral 3 is a lower plate for fixing a test piece, reference numeral 4 is a ring, reference numeral 5 is a test piece, reference numeral 6 is an intermediate plate for fixing a test piece, reference numeral 7 is an upper plate for fixing a test piece, reference numerals 8, 8' are set screws, reference numeral 9 is a base plate, reference numerals 10, 10' are support rods, reference numeral 11 is a screw for fixing an adapter, reference numeral 12 is a test desk, reference numeral 13 is a test desk up-and-down movement slit, reference numeral 14 is an equally spaced scale for moving velocity, reference numeral 21 is a rod-like body, and reference numeral 22 is a sphere-like body. As shown in Figs. 4 to 6, this apparatus comprises the adapter 1 for breaking stress and the adapter 2 for viscosity for breaking the test piece.

The adapter 1 for breaking stress comprises the lower plate 3 for fixing a test piece, the plate 3 having a 30-mm diameter through-hole at a center portion, 40 mm wide, 70 mm long and 5 mm thick, and having fixing means at both ends thereof, the 17-mm internal diameter, 2-mm thick ring 4 fitted onto the hole at the center of the lower plate 3, the test piece 5 (30 mm×30 mm) (refer to Fig. 5 and Fig. 6), the intermediate plate 6 (40-mm wide, 70-mm long, 3-mm thick plasticized polyvinyl chloride plate) for fixing a test piece, which is mounted on the test piece 5 and has a hole with an internal diameter of 16 mm at the center, and the upper plate 7 (40 mm wide, 70 mm long and 3 mm thick) for fixing a test piece, which is placed on the intermediate plate 6 and has a hole with an internal diameter of 30 mm at the center, these being sequentially placed (refer to Fig. 4). The lower plate 3 for fixing a test piece has a concave portion with a diameter of 35 mm at the center thereof in a depth of 2 mm from a surface thereof so that the ring having an external diameter of 35 mm and an internal diameter of 17 mm is fit therein, the concave portion being formed on an upper portion of a through hole with a diameter of 30 mm (refer to Fig. 6). These plates 3, 6 and 7 are fixed with the set screws 8 and 8' at both end portions thereof, supported with the support rods 10 and 10' on the base plate 9, and fixed to the test desk 12 with the screw 11 for fixing the adapter, together with the base plate 9 (refer to Fig. 4). The base plate 9 is a 40-mm wide, 80-mm long, 2-mm thick plate made of stainless steel, and the height from the base plate to a surface of the lower plate 3 for fixing a test piece is 57 mm (refer to Fig. 5). Further, the adapter 2 for viscosity comprises the rod-like body 21, and the 7-mm diameter sphere-like body 22 made of stainless steal attached to a leading end thereof (refer to Fig. 4)

Then, in the measurement of the breaking strength of the test piece, the test desk 12 moves at a constant speed along the test desk up-and-down movement slit 13 shown in Fig. 4, thereby pressing the test piece with the sphere-like body 22 of the adapter 2 for viscosity to break it, and the braking stress thereof is recorded with an attached recorder (not shown) to determine the breaking strength.

### (2) Measuring Method

The film preparation test piece 5 (30 mm×30 mm) was placed on the ring 4 fitted onto the lower plate 3 for fixing a test piece of the adapter 1 for breaking stress, which was fixed to the test desk, and held down with the intermediate plate 6 (40-mm wide, 70-mm long, 3-mm thick plasticized polyvinyl chloride plate) for fixing a test piece, which had the hole with an internal diameter of 16 mm at the center. Further, the test piece 5 was held down with the metal upper plate 7 (40 mm wide, 70 mm long and 3 mm thick) for fixing a test piece, which had the hole with an internal diameter of 30 mm at the center. The test piece 5 was fixed by tightening the set screws 8 and 8' provided at both ends of the upper plate 7 of these stacked plates. Then, the test desk 12 to which the adapter 1 for breaking stress was fixed was allowed to move upward at a speed of 10 cm/min along the test desk up-and-down movement slit 13 to break the test piece with the sphere-like body 22 of the adapter 2 for viscosity. The breaking stress thereof was recorded with the attached recorder. The average value from three tests was determined, and it was taken as the breaking strength. The results are shown in Table 7.

### 2) [Tensile Strength]

(1) A rheometer (manufactured by Rheotech Co., Ltd., Type RT-3020D-CW) was used as a tensile strength measuring apparatus. Fig. 7 is a schematic perspective view showing a tensile strength measuring adapter of a tensile strength measuring apparatus used in the present invention, and Fig. 8 is a schematic perspective view showing a detecting unit of the tensile strength measuring apparatus, to which an upper adapter is attached. In the figures, reference numeral 31 is a tensile strength measuring adapter, reference numeral 32 is an upper adapter for fixing a test piece, reference numeral 33 is a lower adapter for fixing a test piece, reference numeral 34 is the test piece, reference numeral 35 is an upper adapter attaching member, reference numeral 36 is a test desk, reference numeral 37 is an upper adapter set screw, reference numeral 38 is a lower adapter set screw, reference numeral 39 is a test desk up-and-down movement slit, reference numeral 40 is an equally spaced scale for moving velocity, reference numeral 41 is a detecting unit for moving speed, reference numeral 42 is a knob for coarse adjustment of an indicator, and reference numeral 43 is a knob for fine adjustment of an indicator.

The tensile strength measuring adapter 31 of the tensile strength measuring apparatus used in the present invention comprises the upper adapter 32 for fixing a test piece, which is attached to the detecting unit 41 with the interposition of the upper adapter attaching member 35, and the lower adapter 33 for fixing a test piece, which is mounted on the test desk 36, as shown in Fig. 7 and Fig. 8. The test piece 34 is fixed at a constant length between the upper and lower adapters, and the test desk is allowed to move down to the lower portion to break the test piece, thereby measuring the maximum tension of the test piece. The upper adapter 32 for fixing a test piece is movable up and down, and the test piece is fixed at a position providing a desired length by tightening the set screw 37. The lower adapter 33 for fixing a test piece is constituted so as to be able to move the test desk up and down to fix and break the test piece. The position thereof is adjusted so that the length between the upper adapter 32 for fixing a test piece and the lower adapter 33 for fixing the same becomes 7 cm, and the test piece is fixed with a set screw 38. The test desk 36 moves up and down along the test desk up-and-down movement slit 39, and in the measurement of the tensile strength of the test piece, it is allowed to move downward at a constant speed (6 cm/min) . The equally spaced scale 40 for moving velocity is put in the movement slit 39.

As shown in Fig. 8, the attaching member 35 of the upper adapter for fixing a test piece is further equipped with the detecting unit 41. This detecting unit 41 is one for detecting the tensile strength, and detects and records the tensile strength at the time when the test piece is broken. Then, the measurement value is calculated. The detecting unit 41 is provided with the a knob 42 for coarse adjustment of an indicator and a knob 43 for fine adjustment of an indicator, which are ones for adjusting the initial value to 0 in the measurement of the tensile strength. The size of the upper and lower adapter 32 and 33 is 30 mm wide, 30 mm long and 30 mm high.

### (2) Measuring Method

The test piece 34 cut to 15x100 mm is fixed to the upper adapter 32 for fixing a test piece at the position of 1.5 cm from the upper part, and then, the test desk is allowed to move upward. A lower portion of the test piece is fixed to the lower adapter 33 for fixing a test piece, adjusting the length of the test piece 34 fixed to the upper adapter 32 to 7 cm. Then, the test desk 36 is allowed to move downward at a speed of 6 cm/min to break the test piece 34. The maximum tensile force of the test piece 34 until breakage was reached was recorded with an attached recorder, and the measurement value was determined. The average value was determined from three test measurements, and it was taken as the tensile strength. The results are shown in Table 7.

### 3) [Water Disintegration Test]

Dots are marked on four corners of the film preparation test piece with a permanent marker. Four hundred milliliters of purified water previously heated to 25°C is poured as a test solution into a 500-ml beaker, and a stirrer is placed therein, followed by stirring at 100 rpm. Then, the above-mentioned film preparation test piece was floated in the test solution maintained at 25°C±1°C under stirring, and the time taken until the dots previously marked on the four corners with the permanent marker were dispersed was measured. The test was carried out 5 times, and the average value was determined from five tests. The results are shown in Table 7.

### 4) The solubility of the film preparation of the present invention in the oral cavity is within 60 seconds. The solubility was measured as follows.

The film preparation of the present invention was kept in the oral cavity of a normal adult without water, and the time taken until the film preparation was completely disintegrated and dispersed only by the saliva in the oral cavity was measured. The test was carried out twice, and the average value was calculated therefrom.

As for the film preparation of the present invention, the solubility is not particularly specified. However, taking into consideration various conditions such as the appearance of a drug effect and the problem of unpleasant feeling, it is suitably within 60 seconds.

The rapidly soluble film preparations of the present invention are produced by the following methods.

### (1) In the Case of Only Drug Layer (One Layer, not according to the invention)

A drug and an edible polymer, and a saccharide, and a plasticizer if necessary, and further a sweetener and a colorant as needed are added to an appropriate amount of a solvent (water-ethanol solvent), and dissolved with stirring. The solution is spread on a liner film (release film) such as polyester, and dried to prepare a film. It is stamped out to a suitable size to obtain a product. When the drug is not soluble, but dispersible in the solvent, the drug is previously dispersed, and then, the edible polymer is added.

### (2) In the Case of Laminate (Two Layers) of Drug Layer and Support Layer on One Side Thereof

An edible polymer, and a saccharide and a plasticizer if necessary, and further a colorant (for example, titanium oxide) and the like as needed are added to an appropriate amount of a solvent (water-ethanol solvent), and dissolved or dispersed with stirring to prepare a solution for a support layer. Separately, a drug and an edible polymer, and a saccharide and a plasticizer if necessary, and further a sweetener, a flavor and the like as needed are added to an appropriate amount of a solvent (water-ethanol solvent), and dissolved with stirring to prepare a solution for a drug layer. Then, the solution for a support is spread on a liner film such as polyester, and dried to obtain a film having a suitable thickness. The solution for a drug layer is spread thereon so that the thickness including the support layer reaches a desired value, and dried to prepare a film. This is stamped out to a suitable size to obtain a product.

### (3) In the Case of Laminate (Three Layers) of Drug Layer and Support Layers on Both Sides Thereof

A solution for a support layer and a solution for a drug layer are prepared in the same manner as with (2), and a two-layer film is produced in the same manner as described above. The solution for a support layer is spread on a surface of the drug layer on which the support layer is not provided, and dried to prepare a film having a desired thickness. This film is stamped out to a suitable size to obtain a stamped product.

Examples are shown below.

### Examples

### Example 1 (not according to the invention)

To an appropriate amount of an ethanol/water mixed solvent, 30. 0 parts by weight of chlorpheniramine maleate, 10. 0 parts by weight of hydrogenated maltose starch syrup and 60.0 parts by weight of HPMC were added in this order, and dissolved with stirring. After deaeration, the solution was spread on a polyester liner film, and dried to obtain a film having a thickness of 74.2 µm. The resulting film was stamped out to a square, 16 mm each side, to obtain a rapidly soluble film preparation.

### Example 2 (not according to the invention)

To an appropriate amount of an ethanol/water mixed solvent, 15.0 parts by weight of thiamin hydrochloride, 15.0 parts by weight of polyethylene glycol and 70.0 parts by weight of HPMC were added in this order, and dissolved with stirring. After deaeration, the solution was spread on a polyester liner film, and dried to obtain a film having a thickness of 61.8 µm. The resulting film was stamped out to a square, 16 mm each side, to obtain a rapidly soluble film preparation.

### Examples 3 to 5 (not according to the invention)

Rapidly soluble film preparations were obtained according to formulations of Table 1 in the same manner as with Example 2.

**Table 1**

| Name of Component | Example | | |
|---|---|---|---|
| | 3 * | 4 * | 5 * |
| Thiamin Hydrochloride | - | 20.0 | - |
| Famotidine | 20.0 | - | 20.0 |
| HPMC | 70.0 | 60.0 | 60.0 |
| PEG | 10.0 | 20.0 | 20.0 |
| Total | 100.0 | | |

| | | | |
|---|---|---|---|
| *(not according to the invention) | | | |

### Example 6 (not according to the invention)

To an appropriate amount of an ethanol/water mixed solvent, 20.0 parts by weight of thiamin hydrochloride, 10.0 parts by weight of hydrogenated maltose starch syrup and 70.0 parts by weight of HPC were added in this order, and dissolved with stirring. After deaeration, the resulting solution was spread on a polyester liner film, and dried to obtain a film having a thickness of 80.8 µm. The resulting film was stamped out to a square, 16 mm each side, to obtain a rapidly soluble film preparation.

### Example 7 and 10 (not according to the invention)

Rapidly soluble film preparations were obtained according to formulations of Table 2 in the same manner as with Example 6.

**Table 2**

| Name of Component | Total | | | |
|---|---|---|---|---|
| | 7 | 8 | 9 | 10 |
| Thiamin Hydrochloride | - | 20.0 | - | 20.0 |
| Famotidine | 20.0 | - | 20.0 | - |
| HPC | 70.0 | 70.0 | 80.0 | 75.0 |
| Hydrogenated maltose starch svrup | 10.0 | - | - | - |
| PEG | - | 10.0 | - | 5.0 |
| Total | 100.0 | | | |

### Example 11

To an appropriate amount of an ethanol/water mixed solvent, 20.0 parts by weight of hydrogenated maltose starch syrup was added, and dissolved with stirring. Then, 10.0 parts by weight of titanium oxide was added, and dispersed with stirring. Further, 70.0 parts by weight of HPMC was added to prepare a solution for a support layer. Separately, 50.0 parts by weight of chlorpheniramine maleate and 50.0 parts by weight of HPC were added to an appropriate amount of an ethanol/water mixed solvent, and dissolved with stirring to prepare a solution for a drug layer. Then, the solution for a support layer was spread on a polyester liner film, and dried to obtain a film having a thickness of about 15 µm. The solution for a drug layer was spread thereon, and dried to obtain a film having a thickness of about 45 µm. Finally, the solution for a support layer was spread thereon, and dried to obtain a film having a thickness of about 15 µm. The layers were thus laminated to obtain a film having a thickness of 74.6 µm as a whole. The resulting film was stamped out to a square, 16 mm each side, to obtain a rapidly soluble film preparation.

### Examples 12 to 14

Rapidly soluble film preparations were obtained according to formulations of Table 3 in the same manner as with Example 11.

**Table 3**

| Layer | Name of Component | Example | | |
|---|---|---|---|---|
| | | 12 | 13 | 14* |
| Support Layer | HPMC | 70.0 | | |
| | Hydrogenated maltose starch syrup | 20.0 | | |
| | Titanium Oxide | 10.0 | | |
| | Total | 100.0 | | |
| Drug Layer | chlorpheniramine maleate | 40.0 | 40.0 | 40.0 |
| | HPC | 60.0 | - | - |
| | HPMC | - | 50.0 | - |
| | PVP | - | - | 50.0 |
| | PEG | - | 10.0 | 10.0 |
| | Total | 100.0 | | |

| | | | | |
|---|---|---|---|---|
| *(not according to the invention) | | | | |

### Example 15

To an appropriate amount of an ethanol/water mixed solvent, 10.0 parts by weight of hydrogenated maltose starch syrup and 5 parts by weight of PEG were added, and dissolved with stirring. Then, 15.0 parts by weight of titanium oxide was added, and dispersed with stirring. Further, 70.0 parts by weight of HPMC was added to prepare a solution for a support layer. Separately, 50.0 parts by weight of chlorpheniramine maleate and 50.0 parts by weight of HPC were added to an appropriate amount of an ethanol/water mixed solvent, and dissolved with stirring to prepare a solution for a drug layer. Then, the solution for a support layer was spread on a polyester liner film, and dried to obtain a film having a thickness of about 16 µm. The solution for a drug layer was spread thereon, and dried to obtain a film having a thickness of about 50 µm. Finally, the solution for a support layer was spread thereon, and dried to obtain a film having a thickness of about 16 µm. The layers were thus laminated to obtain a film having a thickness of 81.4 µm as a whole. The resulting film was stamped out to a square, 16 mm each side, to obtain a rapidly soluble film preparation.

### Examples 16 to 19

Rapidly soluble film preparations were obtained according to formulations of Table 4 in the same manner as with Example 15.

**Table 4**

| Layer | Name of Component | Example | | | |
|---|---|---|---|---|---|
| | | 16 | 17 | 18 | 19 * |
| Support Layer | HPMC | 70.0 | | | |
| | Hydrogenated maltose starch syrup | 10.0 | | | |
| | Titanium Oxide | 15.0 | | | |
| | PEG | 5.0 | | | |
| | Total | 100.0 | | | |
| Drug Layer | Chlorpheniramine Maleate | 40.0 | 40.0 | 50.0 | 40.0 |
| | HPC | 60.0 | - | - | - |
| | HPMC | - | 50.0 | 40.0 | - |
| | PVP | - | - | - | 50.0 |
| | PEG | - | 10.0 | 10.0 | 10.0 |
| | Total | 100.0 | | | |

| | | | | | |
|---|---|---|---|---|---|
| *(not according to the invention) | | | | | |

### Example 20

To an appropriate amount of an ethanol/water mixed solvent, 10.0 parts by weight of hydrogenated maltose starch syrup and 5 parts by weight of PEG were added, and dissolved with stirring. Then, 15.0 parts by weight of titanium oxide and 70.0 parts by weight of HPMC were added, and dispersed with stirring to prepare a solution for a support layer. Separately, 50.0 parts by weight of chlorpheniramine maleate, 10.0 parts by weight of PEG and 40.0 parts by weight of HPC were added to an appropriate amount of an ethanol/water mixed solvent, and dissolved with stirring to prepare a solution for a drug layer. Then, the solution for a support layer was spread on a polyester liner film, and dried to obtain a film having a thickness of about 11 µm. The solution for a drug layer was spread thereon, and dried to obtain a film having a thickness of about 60 µm. Finally, the solution for a support layer was spread thereon, and dried to obtain a film having a thickness of about 11 µm. The layers were thus laminated to obtain a film having a thickness of 82 µm as a whole. The resulting film was stamped out to a square, 16 mm each side, to obtain a rapidly soluble film preparation.

### Examples 21 to 23

Rapidly soluble film preparations were obtained according to formulations of Table 5 in the same manner as with Example 20.

**Table 5**

| Layer | Name of Component | Example | | |
|---|---|---|---|---|
| | | 21 | 22 | 23 |
| Support Layer | HPMC | 70.0 | | |
| | Hydrogenated maltose starch syrup | 10.0 | | |
| | Titanium Oxide | 15.0 | | |
| | PEG | 5.0 | | |
| | Total | 100.0 | | |
| Drug Layer | Thiamin Hydrochloride | - | 20.0 | - |
| | Famotidine | 40.0 | - | 30.0 |
| | HPC | 60.0 | 70.0 | 70.0 |
| | PEG | - | 10.0 | - |
| | Total | 100.0 | | |

### Example 24 (not according to the invention)

To an appropriate amount of an ethanol/water mixed solvent, 5. 0 parts by weight of chlorpheniramine maleate, 35.0 parts by weight of erythritol, 0.3 part by weight of saccharin sodium, 0.3 part by weight of 1-menthol and 0.1 part by weight of a flavor were added, and dissolved with stirring. Further, 59.3 parts by weight of HPC was added. After deaeration, the solution was spread on a polyester liner film, and dried to obtain a film having a thickness of 34.2 µm. The resulting film was stamped out to a square, 16 mm each side, to obtain a rapidly soluble film preparation.

### Example 25

To an appropriate amount of an ethanol/water mixed solvent, 10.0 parts by weight of maltitol and 5 parts by weight of PEG were added, and dissolved with stirring. Then, 15.0 parts by weight of titanium oxide and 70.0 parts by weight of HPMC were added, and dispersed with stirring to prepare a solution for a support layer. Separately, 5.0 parts by weight of chlorpheniramine maleate, 35.0 parts by weight of maltose, 0.3 part by weight of saccharin sodium, 0.3 part by weight of 1-menthol, 0.16 part of a flavor and 59.24 parts by weight of HPC were added to an appropriate amount of an ethanol/water mixed solvent, and dissolved with stirring to prepare a solution for a drug layer. Then, the solution for a support layer was spread on a polyester liner film, and dried to obtain a film having a thickness of about 17 µm. The solution for a drug layer was spread thereon, and dried to obtain a film having a thickness of about 26 µm. The layers were thus laminated to obtain a film having a thickness of 43.2 µm as a whole. The resulting film was stamped out to a square, 16 mm each side, to obtain a rapidly soluble film preparation.

### Example 26

To an appropriate amount of an ethanol/water mixed solvent, 10.0 parts by weight of hydrogenated maltose starch syrup and 5 parts by weight of PEG were added, and dissolved with stirring. Then, 15.0 parts by weight of titanium oxide and 70.0 parts by weight of HPMC were added, and dispersed with stirring to prepare a solution for a support layer. Separately, 5.0 parts by weight of chlorpheniramine maleate, 35.0 parts by weight of maltose, 0.5 part by weight of saccharin sodium, 0.3 part by weight of 1-menthol, 0.16 part of a flavor and 59.04 parts by weight of HPC were added to an appropriate amount of an ethanol/water mixed solvent, and dissolved with stirring to prepare a solution for a drug layer. Then, the solution for a support layer was spread on a polyester liner film, and dried to obtain a film having a thickness of about 18 µm. The solution for a drug layer was spread thereon, and dried to obtain a film having a thickness of about 50 µm. Finally, the solution for a support layer was spread thereon, and dried to obtain a film having a thickness of about 18 µm. The layers were thus laminated to obtain a film having a thickness of 85.3 µm as a whole. The resulting film was stamped out to a square, 16 mm each side, to obtain a rapidly soluble film preparation.

### Examples 27 to 30 and Comparative Examples 1 to 4

A drug layer was laminated so as to provide a thickness of Table 6 in the same formulation as that of Example 26 to obtain a film having each thickness as a whole. The resulting film was stamped out to a 14x20 mm rectangle to obtain a rapidly soluble film preparation.

**Table 6**

| Layer | Example | | | | Comparative Example | | | |
|---|---|---|---|---|---|---|---|---|
| | 27 | 28 | 29 | 30 | 1 | 2 | 3 | 4 |
| Thickness of Support Layer | 16 | | | | | | | |
| Thickness of Druq Layer | 98 | 148 | 217 | 242 | 272 | 321 | 340 | 408 |
| Thickness of Support Layer | 16 | | | | | | | |
| Thickness of Whole | 129.6 | 180.0 | 248.8 | 273.4 | 304.7 | 352.3 | 371.7 | 439.3 |

The breaking strength, tensile strength, intraoral disintegration time and water disintegration time in each thickness of the film-shaped preparations of Examples and Comparative Examples, along with the reciprocal of the specific surface area of the film-shaped preparations at that time, are shown in Table 7.

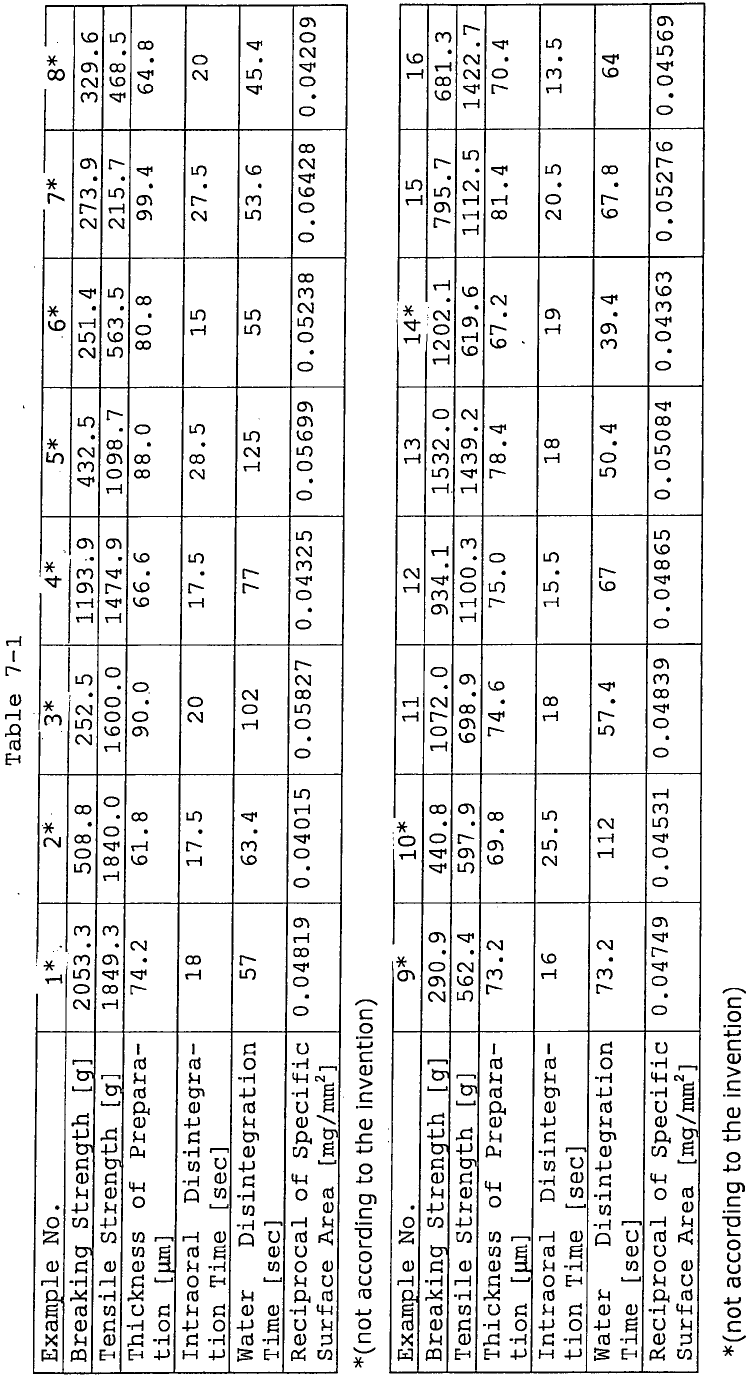

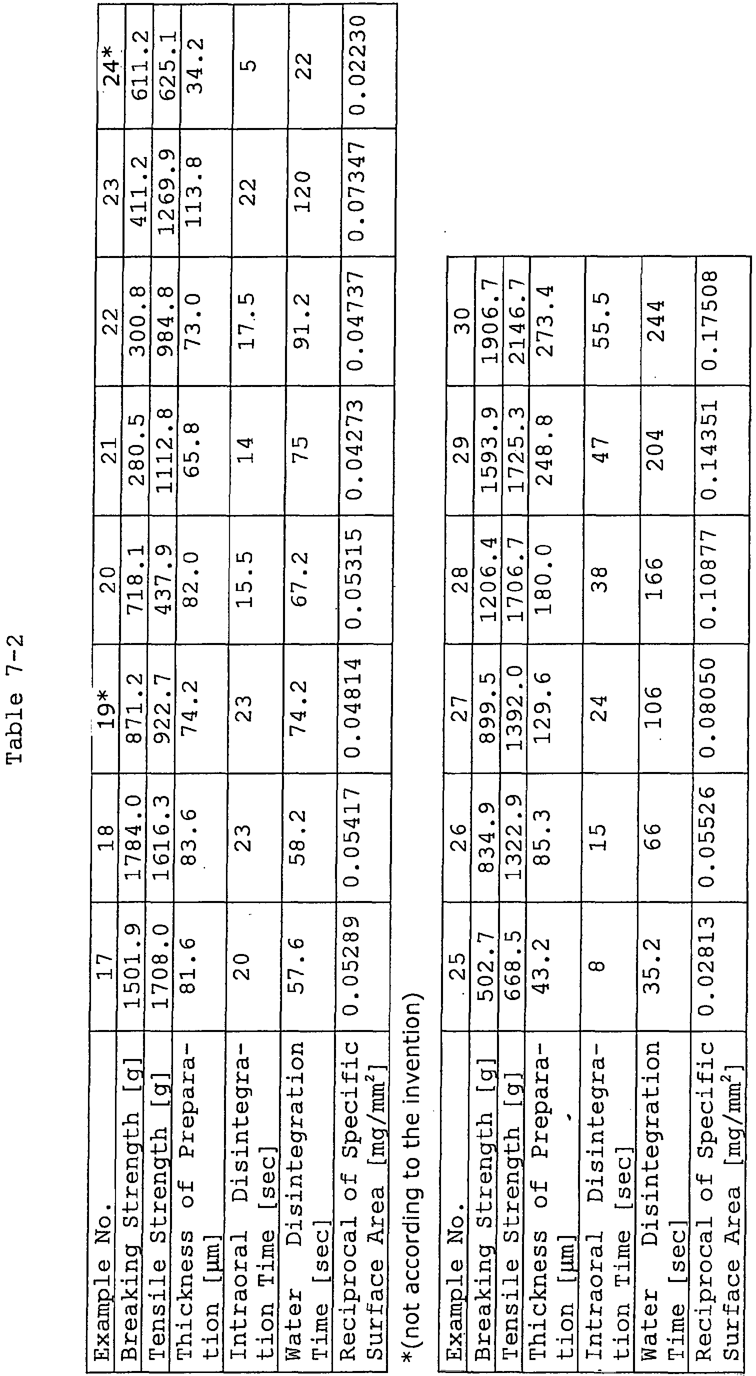

**Table 7-3**

| Comparative Example No. | 1 | 2 | 3 | 4 |
|---|---|---|---|---|
| Breaking Strength [g] | 2472.0 | 3154.7 | 3541.3 | 4333.3 |
| Tensile Strength [g] | 2917.3 | 2842.7 | 3290.7 | 3538.7 |
| Thickness of Preparation [µm] | 304.7 | 352.3 | 371.7 | 439.3 |
| Intraoral Disintegration Time [sec] | 69 | 85 | 85 | 128 |
| Water Disintegration Time [sec] | 330 | 374 | 452 | 516 |
| Reciprocal of Specific Surface Area [mg/mm²] | 0.19213 | 0.22625 | 0.26086 | 0.27619 |

### Advantages of the Invention

The rapidly soluble film preparation of the present invention is easily soluble in the oral cavity even when taken without drinking of water, so that it can be safely administered even to the aged and children. Further, it is also easily taken by a man who lies on his back, so that it can also be administered easily to the bedridden aged and the like. Further, this preparation is in the thin film form, so that it is also convenient to carry about. Furthermore, the rapidly soluble film preparation of the present invention can contain the drug in amounts as considerably large as 25 to 40% by weight based on the whole preparation in the sum of the drug and the saccharide, and therefore, can be effectively used also to the drug of which effective dose is large.

The rapidly soluble film preparation of the present invention is produced by coating. It is therefore unnecessary to add a lubricant, to sprinkle powder sugar or starch on a surface of a sheet-shaped formation for the prevention of blocking, and to conduct surface coating such as sugar coating, as in the conventional extrusion molding. Further, 12-hour ageing at a room temperature of 20°C at a humidity of 50% is not required. Accordingly, it is unnecessary to fear the influence of those lubricants on the preparation, and production operations thereof are extremely simple and also advantageous in cost.

Further, the preparation of the present invention obtained by coating, particularly, one having the support layers on both sides of the preparation, can fully avoid blocking, and can dramatically improve the visual value of the preparation,

The preparation of the present invention obtained by coating requires the drying process. However, the drying time thereof is typically 5 minutes at 60°C. In this case, the use of a material low in heat stability is possible, and it is possible to obtain a preparation without conducting an ageing process as very long as 12 hours.

In the film-shaped preparation of the present invention, the edible polymer (i.e. HPC and/or HPMC) is used as the film forming agent. The edible polymer has film forming ability enough, and further, the film-shaped preparation of the present invention has an appropriate strength. It is therefore unnecessary to particularly thicken the preparation, and it is rapidly (about 60 seconds) soluble in the oral cavity. The breaking strength of the film-shaped preparation of the present invention is from 200 to 3, 000 g/φ7 mm, and the tensile strength of the film is from 200 to 3,000 g/15 mm. Within these ranges, the product is not brittle, and can be used without unpleasant feeling when kept in the mouth.

### Industrial Applicability

The rapidly soluble film preparation of the present invention is easily soluble in the oral cavity even when taken without drinking of water, so that it can be safely administered even to the aged and children. Further, it is also easily taken in the dorsal position (supine position), so that it can also be administered with ease to the bedridden aged and the like. Further, this preparation is in the thin film form, so that it is also convenient to carry about. Furthermore, the rapidly soluble film preparation of the present invention can contain the drug in amounts as considerably large as 25 to 40% by weight based on the whole preparation in the sum of the drug and the saccharide, and therefore, can be effectively used also for the drug having a large effective dosage. The preparation is therefore useful.

## Claims

1. A two- or three-layered film preparation comprising a drug-containing layer and a support layer on one or both side(s) of the drug-containing layer,
wherein both the drug-containing layer and the support layer(s) contain an edible polymer,
wherein the edible polymer is hydroxypropyl cellulose and/or hydroxypropylmethyl cellulose,
wherein the compounding ratio of the drug is from 0.01 to 40% by weight, based on the whole preparation, and the compounding ratio of the edible polymer is from 40 to 99.99% by weight, based on the whole preparation,
wherein the film-shaped preparation has a thickness of from 30 µm to 300 µm, and
wherein the breaking strength of the multi-layered film preparation is from 200 to 3,000 g/φ7 mm, the tensile strength of the film is from 200 to 3,000 g/15 mm, and the preparation is soluble in the oral cavity within 60 seconds.

2. The film preparation according to claim 1,
wherein the edible polymer of the support layer is hydroxypropylmethyl cellulose, the compounding ratio thereof is 50% by weight or more, the edible polymer of the drug-containing layer is hydroxypropyl cellulose, and the compounding ratio thereof is from 40 to 99.99% by weight.

3. The film preparation according to claim 1 or 2, wherein the drug is contained to provide a dose of 0.001 mg to 50 mg, and soluble or dispersible in a water-ethanol solvent.

4. The film preparation according to claim 1, 2 or 3, which further contains a saccharide.

5. The film preparation according to claim 4, wherein the sum of the drug and the saccharide is from 25 to 40% by weight based on the whole preparation.

## Patentansprüche

1. Zwei- oder dreischichtiges Filmpräparat, das eine wirkstoffhaltige Schicht und eine Trägerschicht auf einer oder beiden Seiten der wirkstoffhaltigen Schicht umfasst;
wobei sowohl die wirkstoffhaltige Schicht als auch die Trägerschicht oder -schichten ein essbares Polymer enthalten;
wobei es sich bei dem essbaren Polymer um Hydroxypropylcellulose und/oder Hydroxypropylmethylcellulose handelt;
wobei das Compoundierverhältnis des Wirkstoffs 0,01 bis 40 Gew.-% beträgt, bezogen auf das gesamte Präparat, und das Compoundierverhältnis des essbaren Polymers 40 bis 99,99 Gew.-% beträgt, bezogen auf das gesamte Präparat;
wobei das filmförmige Präparat eine Dicke von 30 µm bis 300 µm aufweist; und
wobei die Reißfestigkeit des mehrschichtigen Filmpräparats 200 bis 3000 g/φ7 mm beträgt, die Zugfestigkeit des Films 200 bis 3000 g/15 mm beträgt und das Präparat in der Mundhöhle innerhalb von 60 Sekunden löslich ist.

2. Filmpräparat gemäß Anspruch 1, wobei es sich bei dem essbaren Polymer der Trägerschicht um Hydroxypropylmethylcellulose handelt, deren Compoundierverhältnis 50 Gew.-% oder mehr beträgt, es sich bei dem essbaren Polymer der wirkstoffhaltigen Schicht um Hydroxypropylcellulose handelt und deren Compoundierverhältnis 40 bis 99,99 Gew.-% beträgt.

3. Filmpräparat gemäß Anspruch 1 oder 2, wobei der Wirkstoff in einer solchen Menge enthalten ist, dass man eine Dosis von 0,001 mg bis 50 mg erhält, und in einem Wasser-Ethanol-Lösungsmittel löslich oder dispergierbar ist.

4. Filmpräparat gemäß Anspruch 1, 2 oder 3, das weiterhin ein Saccharid enthält.

5. Filmpräparat gemäß Anspruch 4, wobei die Summe des Wirkstoffs und des Saccharids 25 bis 40 Gew.-% beträgt, bezogen auf das gesamte Präparat.

## Revendications

1. Préparation sous forme de film à deux ou trois couches comprenant une couche contenant du médicament et une couche de support sur un ou sur les deux côtés de la couche contenant du médicament,
dans laquelle la couche contenant du médicament et la ou les couche(s) de support contiennent toutes deux un polymère comestible,
dans laquelle le polymère comestible est l'hydroxypropylcellulose et/ou l'hydroxypropylméthylcellulose,
dans laquelle le rapport de mélange du médicament est de 0,01 à 40% en poids, par rapport à la préparation totale, et le rapport de mélange du polymère comestible est de 40 à 99,99% en poids, par rapport à la préparation totale,
dans laquelle la préparation sous forme de film a une épaisseur de 30 µm à 300 µm, et
dans laquelle la résistance à la rupture de la préparation sous forme de film multicouche est de 200 à 3000 g/φ7 mm, la résistance à la traction du film est de 200 à 3000 g/15 mm, et la préparation est soluble dans la cavité orale en moins de 60 secondes.

2. Préparation sous forme de film selon la revendication 1,
dans laquelle le polymère comestible de la couche de support est l'hydroxypropylméthylcellulose, son rapport de mélange est de 50% en poids ou plus, le polymère comestible de la couche contenant le médicament est l'hydroxypropylcellulose, et son rapport de mélange est de 40 à 99,99% en poids.

3. Préparation sous forme de film selon la revendication 1 ou 2, dans laquelle le médicament est présent pour fournir une dose de 0,001 mg à 50 mg, et est soluble ou dispersible dans un solvant eau-éthanol.

4. Préparation sous forme de film selon la revendication 1, 2 ou 3, qui contient en outre un saccharide.

5. Préparation sous forme de film selon la revendication 4, dans laquelle la somme du médicament et du saccharide est de 25 à 40% en poids par rapport à la préparation totale.
